# EUROPEAN PATENT APPLICATION

(11) **EP 4 461 819 A1**
(43) Date of publication of application: **13.11.2024**
(21) Application number: 22919080.6
(22) Date of filing: 23.12.2022
(51) Int. Cl.: C12Q 1/6848, C12N 9/24, C12N 15/52, C12N 15/70, C07K 1/16

(54) **METHOD FOR REMOVING CARRYOVER CONTAMINATION IN NUCLEIC ACID AMPLIFICATION REACTIONS BY USING PHOTOBACTERIUM LEIOGNATHI-DERIVED URACIL-DNA GLYCOSYLASE**

(30) Priority: 04.01.2022 KR 20220001058
(71) Applicant: Seegene, Inc., Seoul 05548 (KR)
(72) Inventor: KWON, Suk-Tae, Seoul 05764 (KR); NA, Hyewon, Guri-si Gyeonggi-do 11939 (KR); CHO, Sung Suk, Seoul 05240 (KR); KWON, Chanho, Seoul 05532 (KR)
(74) Representative: Kilger, Ute
(86) International application number: PCT/KR2022/021237
(87) International publication number: WO 2023/132543

(57) **Abstract**

The present invention relates to uracil-DNA glycosylase (UDG) derived from *Photobacterium leiognathi* and to the use thereof. The novel UDG of the present invention is an enzyme isolated from *Photobacterium leiognathi,* which is a psychrophilic bacterium, and is characterized by having an activity of degrading uracil bases from uracil-containing DNA substrate and being easily denatured at low temperatures. Accordingly, the UDG of the present invention can be effectively used to eliminate carryover contamination occurring during the nucleic acid amplification reaction, resulting in increased accuracy (elimination of false positives), purity and amplification efficiency in nucleic acid amplification reactions.

## Description

### CROSS-REFERENCE TO RELATED APPLICATIONS

This application claims priority from Korean Patent Application No. 2022-0001058, filed on January 4, 2022, in the Korean Intellectual Property Office, the disclosure of which is incorporated herein by reference in its entirety.

### BACKGROUND OF THE INVENTION

### FIELD OF THE INVENTION

The present invention relates to a method for eliminating carryover contamination in a nucleic acid amplification reaction using uracil-DNA glycosylase derived from *Photobacterium leiognathi,* to a method for producing the UDG, and to the UDG produced by the method.

### DESCRIPTION OF THE RELATED ART

Uracil-DNA glycosylase (hereinafter referred to as "UDG") is known as an enzyme which repairs damaged DNA by recognizing the damaged base in DNA and hydrolyzing N-glycosidic bond between the uracil base and the deoxyribose sugar in DNA, thereby eliminating the damaged base from DNA. UDG has been first isolated from *E. coli,* and then found in various bacteria including Bacillus. UDG has a molecular weight of about 25 to 35 kDa and is known to specifically select and eliminate only uracil bases, among other bases, from DNA (see Lindahl, T., Proc. Natl. Acad. Sci. USA 71, 3649-3653, 1974; Cone, R. et al., Biochemistry 16, 3194-3201, 1977).

Uracil is a base of RNA but is sometimes found in DNA, which occurs, when uracil generated by naturally occurring deamination of cytosine is inserted into DNA, or when dUTP, instead of dTTP, is accidentally inserted into DNA during DNA replication process. However, UDG specifically eliminates uracil bases in DNA, not uracil bases in RNA, thus forming an apyrimidinic site (AP) where a base is eliminated, and facilitating actions of various DNA repair enzymes such as AP endonuclease, DNA polymerase, and DNA ligase or the like, to repair damaged or mutated DNA (see Chen, R. et al., J Gen Virol. 83, 2339-2345, 2002; Lanes, O. et al., Extremophiles6, 73-86, 2002).

Meanwhile, nucleic acid amplification reactions including polymerase chain reactions (PCR) and isothermal amplification reactions are techniques for isolating or identifying useful genes by amplifying a specific nucleic acid region in large quantities *in vitro* using DNA polymerase (see Erlich, H. A., J Clin Immunol 9, 437-447, 1989; Shin, H. J. et al., J Microbiol Biotechnol 15, 1359-136, 2005). The nucleic acid amplification reactions have significantly contributed to lowering the detection limit required for detection of nucleic acids, and is currently used for detection and identification of diseases such as diagnosis of viruses, pathogenic bacteria, and the like. However, it has disadvantages that it is difficult to detect very low concentrations of nucleic acids and the reaction efficiency varies depending on the reactor. In particular, one of the most significant problems of these techniques in clinical diagnosis is contamination of a sample, which may cause false positives after a nucleic acid amplification reaction. Among others, carryover contamination, in which any residual products of previous nucleic acid amplification reactions affect next nucleic acid amplification reactions, is the most problematic (see Sobek, H. et al., FEBS Lett 388, 1996). The amplification of the residual products may lead to false-positive results even in the absence of target nucleic acids.

In order to prevent carryover contamination occurring during the nucleic acid amplification reactions, a method of performing PCR using dUTP instead of dTTP has been disclosed by Longo *et al.* (see Longo, M. C. et al., Gene 93, 125-128, 1990). Further, there have been suggested methods comprising, prior to a nucleic acid amplification reaction, treating a template DNA with UDG to eliminate a very small amount of contaminated uracil-DNA in a sample, heating the mixture to inactivate the UDG, and adding dUTP instead of dTTP to carry out the nucleic acid amplification reaction (see Udaykumar., et al., Nucleic Acids Res. 21, 3917-3918, 1993; Taggart et al., J. Virol. Methods 105, 57-65, 2002). Therefore, products that treat or include UDG in a nucleic acid amplification reaction have been currently commercially available.

Mesophilic UDGs, including UDGs from *E. coli,* have the disadvantage of not being easily inactivated at a high temperature of 60°C or more but maintaining some of their activity so that the uracil-containing DNA products which have been amplified in a nucleic acid amplification reaction using dUTP undergo degradation, resulting in reduced amount of final products. For example, as a reverse transcription step using reverse transcriptase is usually performed at about 55°C to about 60°C, which corresponds to a limiting temperature for the action of reverse transcriptase, in order to unwind the secondary structure of RNA reaction, the remaining undenatured mesophilic UDG may result in significantly reduced amount of the final products of the reaction to which dUTP is added. Therefore, in order to remove DNA containing contaminated dUMP, it is necessary to inactivate UDG after treatment with UDG and then perform a nucleic acid amplification reaction using dUTP again.

Recently, there has been growing interest in developing heat-labile, psychrophilic UDGs that allow nucleic acid amplification reactions to be performed immediately after UDG treatment without an additional heating step for deactivating UDGs.

To this end, the present inventors have endeavored to develop psychrophilic UDGs. As a result, the present inventors have isolated novel UDG from *Photobacterium leiognathi,* a psychrophilic bacterium, and confirmed that the UDG has stable enzyme activity at 25°C and is easily inactivated at 50°C.

### SUMMARY OF THE INVENTION

It is an object of the present invention to provide a method for eliminating carryover contamination in a nucleic acid amplification reaction using novel uracil-DNA glycosylase (UDG).

It is another object of the present invention to provide a method for producing the UDG.

It is another object of the present invention to provide the UDG, a nucleic acid molecule comprising a nucleotide sequence encoding the same, a recombinant vector comprising the nucleic acid molecule, a host cell comprising the recombinant vector, and a composition for nucleic acid amplification reaction, comprising the UDG.

The present inventors have attempted to address the problems caused by the use of conventional uracil-DNA glycosylase (UDG), and to provide heat-labile, psychrophilic UDG that allows nucleic acid amplification reactions to be performed immediately after UDG treatment without an additional heating step for deactivating UDG. Accordingly, the present inventors have developed novel UDG from *Photobacterium leiognathi,* which is a psychrophilic bacterium, and confirmed that the UDG is effective in eliminating carryover contamination in nucleic acid amplifications.

In an aspect of the present invention, there is provided a method for eliminating carryover contamination in a nucleic acid amplification reaction, comprising the step of incubating a composition for the nucleic acid amplification reaction with a sample suspected of containing a nucleic acid, wherein the composition comprises uracil-DNA glycosylase (UDG) derived from *Photobacterium leiognathi.*

In an embodiment, the UDG comprises the amino acid sequence represented by SEQ ID NO: 2.

In an embodiment, the UDG is encoded by the nucleotide sequence represented by SEQ ID NO: 1.

In an embodiment, the incubating is performed at 10 to 25°C.

In an embodiment, the nucleic acid amplification reaction is a polymerase chain reaction (PCR) or an isothermal amplification reaction.

In another aspect of the present invention, there is provided a method for producing uracil-DNA glycosylase (UDG) derived from *Photobacterium leiognathi,* comprising the steps of: (a) culturing a host cell transformed to express UDG derived from *Photobacterium leiognathi;* (b) obtaining the UDG in the form of inclusion bodies from the cultured host cell; (c) refolding the resulting UDG in the form of inclusion bodies; and (d) purifying the refolded UDG.

In an embodiment, the UDG comprises the amino acid sequence represented by SEQ ID NO: 2.

In an embodiment, the UDG is encoded by the nucleotide sequence represented by SEQ ID NO: 1.

In an embodiment, the host cell is *Escherichia coli.*

In an embodiment, the host cell is transformed with an expression vector comprising the nucleotide sequence represented by SEQ ID NO: 1.

In an embodiment, the expression vector comprises a T7 promoter.

In an embodiment, the culturing of step (a) is performed in the presence of IPTG.

In an embodiment, the UDG in the form of inclusion bodies in step (a) is obtained by lysing the host cell.

In an embodiment, the method further comprises washing the resulting inclusion bodies after step (a).

In an embodiment, the washing is performed using a buffer containing urea, Tris-HCl, NaCl, Triton X-100, or a combination thereof.

In an embodiment, the method further comprises solubilizing the resulting inclusion bodies after step (a).

In an embodiment, the solubilizing is performed using a buffer containing Tris-HCl, NaCl, imidazole, guanidine hydrochloride, 2-mercaptoethanol, or a combination thereof.

In an embodiment, the refolding of step (b) is performed on a column in column chromatography.

In an embodiment, a urea gradient from high concentration to low concentration is applied on the column in column chromatography.

In another aspect, there is provided uracil-DNA glycosylase (UDG) derived from *Photobacterium leiognathi,* comprising the amino acid sequence represented by SEQ ID NO: 2.

In an embodiment, the UDG has optimal activity at 10 to 25°C.

In an embodiment, the UDG is inactivated at 50°C or higher.

In another aspect, there is provided a nucleic acid molecule comprising a nucleotide sequence encoding the UDG.

In an embodiment, the nucleic acid molecule comprises the nucleotide sequence represented by SEQ ID NO: 1.

In another aspect, there is provided a recombinant vector comprising the nucleic acid molecule.

In another aspect, there is provided a host cell comprising the recombinant vector.

In another aspect, there is provided a composition for a nucleic acid amplification reaction, which comprises the UDG.

### I. Uracil-DNA glycosylase of photobacterium leiognathi

According to an aspect of the present invention, there is provided uracil-DNA glycosylase (UDG) derived from *Photobacterium leiognathi,* comprising the amino acid sequence represented by SEQ ID NO: 2.

The UDG may be obtained from a variety of conventionally known strains of *Photobacterium leiognathi. Photobacterium leiognathi* is a species of Gram-negative bacteria that is bioluminescent and a symbiont of ponyfish (Leiognathidae).

The *Photobacterium leiognathi* strain from which the UDG of the present disclosure may be derived may be a naturally occurring strain or a commercially available strain.

As an example, the *Photobacterium leiognathi* strains from which the UDG of the present disclosure may be derived include, but are not limited to, ATCC 25521, ATCC 33469, ATCC 11040, ATCC 33979, ATCC 27561, ATCC 25587, ATCC 33981, ATCC 33980, ATCC 33982 and ATCC 33510, all of which are available from American Type Culture Collection (ATCC).

In certain embodiments, the *Photobacterium leiognathi* strain from which the UDG of the present disclosure may be derived is *Photobacterium leiognathi* ATCC 33980.

In an embodiment, the UDG includes a polypeptide having the amino acid sequence represented by SEQ ID NO: 2 and functional equivalents thereof, as will be appreciated by those skilled in the art. The term "functional equivalent" refers to a polypeptide which has at least 80%, more preferably at least 90% sequence homology with an amino acid sequence as a result of amino acid mutation (insertion, substitution, or deletion), and which exhibits substantially the same physiological activity as the UDG protein of the present invention. As used herein, the term "substantially the same physiological activity" refers to an activity of specifically degrading uracil bases in DNA.

Such amino acid mutation is made based on the relative similarity of the amino acid side-chain substituents, e.g., hydrophobicity, hydrophilicity, charge, size, etc. Analysis of the size, shape, and type of amino acid side-chain substituents reveals that arginine, lysine, and histidine are all positively charged residues; alanine, glycine, and serine are of similar sizes; and phenylalanine, tryptophan, and tyrosine are of similar shapes. Thus, in view of that, arginine, lysine, and histidine; alanine, glycine, and serine; and phenylalanine, tryptophan, and tyrosine can be said to be biologically functional equivalents.

When introducing the amino acid mutation, hydropathic indices of amino acids may be considered. Each amino acid is assigned a hydropathic index according to its hydrophobicity and charge: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The hydropathic indices of amino acids are very important in giving an interactive biological function to a protein. It is a known fact that substitution of an amino acid with other amino acid having a similar hydropathic index can retain similar biological activity. When the mutation is introduced with reference to the hydropathic index, substitution is preferably made between amino acids showing a difference in hydropathic index within ±2, more preferably within ±1, and even more preferably within ±0.5.

It is also known that substitutions between amino acids having similar hydrophilicity values result in proteins with equivalent biological activity. As disclosed in U.S. Pat. No. 4,554,101, the following hydrophilic values are assigned to each amino acid residue: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5±1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

When mutations are introduced with reference to hydrophilicity values, substitution is preferably made between amino acids showing a difference in hydrophilicity value within ±2, more preferably within ±1, and even more preferably within ±0.5.

Amino acid exchange in proteins that do not alter the activity of the molecule as a whole is known in the art (see H. Neurath, R. L. Hill, The Proteins, Academic Press, New York, 1979). The most commonly occurring exchanges are those between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly.

The UDG of the present invention encompasses UDGs containing minor changes in the amino acid sequences described above, i.e., modifications that have little effect on the tertiary structure and the function of the enzyme. Thus, in certain embodiments, they may have at least 90%, 93%, 95% or 98% similarity even if they do not match the sequences described above.

In an embodiment, the UDG has optimal activity at a temperature of 10 to 25°C, but it is not limited thereto.

In an embodiment, the UDG is inactivated at a temperature of 50°C or higher.

Therefore, the UDG enzyme of the present invention is characterized by being easily inactivated at a temperature of 50°C or higher compared to the most frequently used UDG enzymes from *E. coli* and other known UDG enzymes, all of which retain activity even at a high temperature of 60°C or higher.

As shown in the Examples of the present invention, the UDG enzyme of the present invention is heat-labile, and thus loses its activity even when treated at 50°C for 2 minutes or more.

### II. Nucleic acid molecule

According to another aspect of the invention, there is provided a nucleic acid molecule comprising a nucleotide sequence encoding the UDG as described above.

As used herein, the term "nucleic acid molecule" is meant to comprehensively include DNA (gDNA and cDNA) and RNA molecules, and a nucleotide as a basic structural unit in the nucleic acid molecule includes natural nucleotides as well as sugar or base-modified analogs (see Scheit, Nucleotide Analogs, John Wiley, New York (1980); Uhlman and Peyman, Chemical Reviews, 90: 543-584 (1990)).

The nucleic acid molecule may be isolated from nature or prepared by chemical synthesis. The nucleic acid molecule may preferably be isolated from a psychrophilic bacterium, and more preferably, from *Photobacterium leiognathi.*

In one embodiment, the nucleic acid molecule comprises the nucleotide sequence represented by SEQ ID NO: 1.

It is obvious to those skilled in the art that the nucleotide sequence encoding the UDG of the present invention is any nucleotide sequence encoding an amino acid sequence constituting the UDG, and is not limited to any specific nucleotide sequence.

This is because the occurrence of a mutation in the nucleotide sequence may not result in a change in the protein sequence when the mutated nucleotide sequence is expressed as a protein. This is called codon degeneracy. Thus, the nucleotide sequence comprises one comprising a functionally equivalent codon, a codon encoding the same amino acid (e.g., there are six codons for arginine or serine, based on codon degeneracy), or a codon encoding a biologically equivalent amino acid.

The nucleic acid molecule encoding the UDG of the present invention is deemed to comprise a nucleotide sequence which shows substantial identity to the nucleotide sequence described above. The substantial identity means a nucleotide sequence having at least 80% homology, more preferably at least 90% homology, and most preferably at least 95%, 97%, 98%, or 99% homology when the nucleotide sequence of the present invention is aligned with any other sequence for maximum match and the aligned sequences are analyzed by one of algorithms commonly used in the art.

Considering mutants having the biologically equivalent activity as described above, it is deemed that the nucleic acid molecule encoding the UDG of the present invention also encompasses a sequence exhibiting substantial identity to the sequence described in the sequence listing. The substantial identity means a sequence having at least 61% homology, more preferably 70% homology, even more preferably 80% homology, and most preferably 90% homology when the sequence of the present invention is aligned with any other sequence for maximum match and the aligned sequences are analyzed by one of algorithms commonly used in the art. Alignment methods for sequence comparison are known in the art. Various methods and algorithms for alignment are disclosed in Smith and Waterman, Adv. Appl. Math. 2:482(1981); Needleman and Wunsch, J. Mol. Bio. 48:443(1970*);* Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992) and Pearson et al., Meth. Mol. Biol. 24:307-31(1994). NCBI Basic Local Alignment Search Tool (BLAST) (see Altschul et al., J. Mol. Biol. 215:403-10(1990)) is available from the National Center for Biological Information (NBCI) and can be used in conjunction with sequence analysis programs such as blastp, blastn, blastx, tblastn and tblastx on the Internet. BLAST is accessible via the BLAST page at the ncbi website. The sequence homology comparison method using this program can be found on the BLAST help page at the ncbi website.

### III. Recombinant vector

According to another aspect of the present invention, there is provided a recombinant vector comprising the nucleic acid molecule as describe above.

As used herein, the term "recombinant vector" refers to a vector into which a nucleic acid molecule encoding the UDG is incorporated and may be used interchangeably with "expression vector" when intended to express the UDG in a cell.

The recombinant vector or expression vector herein may be one of various vectors known in the art.

The vector may include various components for expression of the UDG.

In an embodiment, the vector comprises a promoter.

As used herein, the term "promoter" refers to a nucleic acid sequence encoding an amino acid that includes a binding site for RNA polymerase and has the activity of initiating transcription of a downstream gene into mRNA. The promoter may be operably linked to the UDG. The promoter as used herein encompasses various promoters known to be capable of driving transcription of a protein of interest in a host cell. The promoter may be an inducible promoter or a constitutive promoter depending on the expression pattern of the protein of interest.

As used herein, the term "operably linked" refers that a fragment is arranged such that transcription is initiated by the promoter and proceeds through the amino acid coding sequence to the termination code.

The vector of the present invention may be constructed for prokaryotic cells or eukaryotic cells as a host.

For example, when the vector of the present invention is an expression vector and employs a prokaryotic cell as a host, the vector generally includes a strong promoter capable of transcription initiation (e.g., pL^{λ} promoter, *trp* promoter, *lac* promoter, T7 promoter, *tac* promoter, etc.), a ribosome binding site for translation initiation, and a transcription/translation terminator sequence. When *E. coli* is used as a host cell, a promoter and an operator site of the tryptophan biosynthesis pathway of *E coli* (Yanofsky, C., J. Bacteriol., 158: 1018-1024 (1984)) and a leftward promoter of phage λ (pL^{λ} promoter, Herskowitz, I. and Hagen, D., Ann. Rev. Genet., 14: 399-445 (1980)) may be used as a regulatory site.

Meanwhile, the vector available in the present invention may be prepared by manipulating a plasmid (e.g., pSK349, pSC101, ColE1, pBR322, pUC8/9, pHC79, pGEX series, pET series, pUC19, etc.), a phage (e.g., λgt˙λ4B, λ-Charon, λΔz1, M13, etc.), or a virus (e.g., SV40, etc.), which are often used in the art.

Meanwhile, when the vector of the present invention is an expression vector and a eukaryotic cell is used as a host, a promoter derived from a genome of a mammalian cell (e.g., a metallothionein promoter) or a promoter derived from a mammalian virus (e.g., an adenovirus late promoter, a vaccinia virus 7.5K promoter, a SV40 promoter, a cytomegalovirus promoter, and a tk promoter of HSV) may be used, and generally has a polyadenylation sequence as a transcription terminator sequence.

In one embodiment, the vectors used herein may further comprise a transcription terminator. The transcription terminator is essential for processing and polyadenylation of messenger RNA.

In an embodiment, the vector of the present invention may further comprise a sequence for facilitating purification of UDG. Examples of sequences for facilitating the purification include, but are not limited to, glutathione S-transferase (Pharmacia, USA), maltose binding protein (NEB, USA), FLAG (IBI, USA) and 6x His (hexahistidine; Qiagen, USA).

In a specific embodiment, the vector used herein further comprises a nucleotide sequence encoding a histidine tag at the terminus of the UDG. The histidine tag is used to facilitate purification of a protein using affinity chromatography. Tagging histidine to the terminus of the protein of interest significantly increases the affinity of the protein for metal ions, allowing for easy purification. Contacting a protein having a histidine tag with a column onto which metal ions such as nickel are immobilized under conditions of pH 8.0 or higher allows the histidine tag to chelate the metal ions and bind to the column, thereby recovering a target protein with high purity.

The nucleotide sequence encoding the histidine tag may be linked to the C-terminus or N-terminus of the UDG. In certain embodiments, the nucleotide sequence encoding the histidine tag is linked to the C-terminus of the UDG.

The histidine tag may consist of at least 6 histidines. In one embodiment, the histidine tag consists of 6 histidines (hexahistidine). In another embodiment, the histidine tag consists of 7 histidines (heptahistidine). In another embodiment, the histidine tag consists of 8 histidines (octahistidine). In another embodiment, the histidine tag consists of 9 histidines (nonahistidine). In another embodiment, the histidine tag consists of 10 histidines (decahistidine). The number of histidines that make up the histidine tag can be easily adjusted by those skilled in the art.

The vectors used herein further comprise an origin of replication.

In addition, the vector used herein further comprises a multiple cloning site (MCS).

In addition, the vector used herein may include an antibiotic resistance gene commonly used in the art as a selection marker, for example, resistance genes against ampicillin, gentamicin, carbenicillin, chloramphenicol, streptomycin, kanamycin, geneticin, neomycin, and tetracycline.

Optionally, the vector may further carry a gene encoding a reporter molecule (e.g., luciferase and β-glucuronidase).

The vector system of the present invention may be constructed by various methods known in the art, and a specific method is disclosed in Sambrook, et al., Molecular Cloning, A Laboratory Manual, Cold Spring Harbor Laboratory Press (2001), which is incorporated herein by reference.

In certain embodiments, the vector of the present invention is pET-22b-based expression vectors for expression in *E. coli.* The pET-22b-based expression vector refers to a pET-22b expression vector known in the art or a modified expression vector thereof.

An example of the pET-22b-based expression vector is the pTE-22b(+) vector, which has an N-terminal *pelB* signal sequence for potential periplasmic localization and a C-terminal histidine tag for purification. In addition, the pTE-22b(+) vector has a T7 promoter, a T7 terminator, and a β-lactamase gene (Bla) for ampicillin resistance as a selection marker.

The present inventors has constructed a recombinant vector by inserting a *Nde*I- and XhoI-treated fragment of the UDG enzyme of *Photobacterium leiognathi* into a pET-22b expression vector (Novagen, USA) treated with the same restriction enzymes. The constructed recombinant vector is referred to herein as "pET-22b *Ple* UDG". The constructed recombinant vector was then transformed into *E. coli* BL21 (DE3).

### IV. Host cells

According to another aspect of the present invention, there is provided a host cell comprising the recombinant vector. The host cell is obtained by transformation with the recombinant vector.

The host cell capable of stably and continuously cloning and expressing the vector of the present invention may be any one known in the art, including, but are not limited to, *E.* colistrains such as *E. coli* Origami2, *E. coli* JM109, *E. coli* BL21(DE3), *E. coli* RR1, *E. coli* LE392, *E. coli* B*, E. coliX* 1776, and *E. coli* W3110, *Bacillus* strains such as *Bacillus subtilis* and *Bacillus thuringiensis,* and Enteric pathogens and strains such as *Salmonella typhimurium, Serratia marcescens,* and various *Pseudomonas* species*.*

In addition, when the vector of the present invention is transformed into eukaryotic cells, yeast (Saccharomyces cerevisiae), insect cells and animal cells (e.g., Chinese hamster ovary (CHO) cell line, W138, BHK, COS-7, 293, HepG2, 3T3, RIN and MDCK cell line) may be used as host cells.

When the host cell is prokaryotic, transforming the vector of the present invention into the host cell may be performed by CaCl₂ method (Cohen, S.N. et al., Proc. Natl. Acac. Sci. USA, 9: 2110-2114(1973)), Hanahan method (Cohen, S.N. et al., Proc. Natl. Acac. Sci. USA, 9:2110-2114(1973)); and Hanahan, D., J. Mol. Biol., 166:557-580(1983)) and Electroporation method (Dower, W.J. et al., Nucleic. Acids Res., 16:6127-6145(1988)). In addition, when the host cell is eukaryotic, the vector may be injected into the host cell by microinjection method (Capecchi, M.R., Cell, 22:479 (1980)), calcium phosphate precipitation method (Graham, F.L. et al., Virology, 52:456 (1973)), electroporation method (Neumann, E. et al., EMBO J., 1:841 (1982)), liposome-mediated transfection method (Wong, T.K. et al., Gene, 10:87 (1980)), DEAE-dextran treatment method (Gopal, Mol. Cell Biol., 5:1188-1190(1985)), and Gene bombardment (Yang et al., Proc. Natl. Acad. Sci., 87:9568-9572 (1990)), or the like.

The recombinant vector injected into the host cell herein can express the recombinant UDG in the host cell. For example, when the expression vector comprises a lac promoter, the expression of the UDG gene is inducible by treatment of a host cell with IPTG.

The culture of the transformed host cell may be performed by a known host cell culture method or a modified version thereof. For example, when the host cell is *E. coli,* a medium for culturing the transformed host cell may be a natural medium or a synthetic medium if it includes a carbon source, a nitrogen source, an inorganic salt, and the like, which may be efficiently used by *E. coli.* The carbon source that can be used includes carbohydrates such as glucose, fructose, and sucrose; starch and its hydrolysates; organic acids such as acetic acid and propionic acid; alcohols such as ethanol, propanol, and glycerol, and the like. The nitrogen source includes ammonia; ammonium salts of inorganic or organic acids such as ammonium chloride, ammonium sulfate, ammonium acetate, and ammonium phosphate; peptone, meat extract, yeast extract, corn steep liquor, casein hydrolysate, soybean extract, soybean hydrolysate; various fermented cells and their degraded products. The inorganic salt includes potassium dihydrogen phosphate, dipotassium hydrogen phosphate, magnesium phosphate, magnesium sulfate, sodium chloride, manganese sulfate, copper sulfate, calcium carbonate, and the like.

The culturing is usually performed under aerobic conditions such as by shaking culture or rotation by a rotator. The temperature for culturing is preferably in the range of from 10 to 40°C and the time period for culturing is typically from 5 hours to 7 days. The pH of the medium is preferably maintained in the range of 3.0 to 9.0 during culture. The pH of the medium may be adjusted with inorganic or organic acids, alkali solutions, urea, calcium carbonate, ammonia, etc. Antibiotics such as ampicillin, streptomycin, chloramphenicol, kanamycin, and tetracycline may be added, if necessary, for the maintenance and expression of the recombinant vector during culture. When culturing a host cell transformed with a recombinant expression vector having an inducible promoter, a suitable inducer may be added to the medium if necessary. For example, when the expression vector contains a lac promoter, IPTG (isopropyl-beta-D-thiogalactopyranoside) may be added, and when the expression vector contains a trp promoter, indoleacrylic acid may be added to the medium.

### V. Composition for nucleic acid amplification reaction

According to another aspect, there is provided a composition for a nucleic acid amplification reaction, which comprises the uracil-DNA glycosylase (UDG).

The composition for a nucleic acid amplification reaction may comprise the UDG of the present invention in a concentration of, for example, 0.5 to 10 units.

In addition, the composition for a nucleic acid amplification reaction may typically contain components used for amplifying a specific nucleic acid region in large quantities *in vitro,* for example, a polymerase, different nucleotide triphosphates (dNTPs), a primer capable of binding to and amplifying the specific nucleic acid region, a probe, and an appropriate buffer. The polymerase may be DNA polymerase, RNA polymerase, or reverse transcriptase. The primer refers to a single-stranded oligonucleotide that can act as an initiation point for template-directed DNA synthesis under appropriate conditions and temperatures in an appropriate buffer. The probe refers to a single-stranded or double-stranded oligonucleotide that binds to the specific nucleic acid region to generate a signal indicative of its presence.

The components other than UDG that may be included in the composition for nucleic acid amplification reaction are known in the art, and may be easily adjusted by those skilled in the art according to the nucleic acid to be amplified and detected.

In one embodiment, the dNTPs comprise dUTP instead of dTTP.

In one embodiment, the nucleic acid amplification reaction is a polymerase chain reaction (PCR) or an isothermal amplification reaction.

The PCR reaction may be any type of PCR reaction known in the art, and may encompass a direct PCR in which an enzyme, a substrate, and a PCR composition are added at once to perform an enzymatic reaction, and a reverse transcriptase-PCR reaction using reverse transcriptase.

The isothermal amplification reaction may be any of isothermal amplification reactions known in the art, for example, including rolling circle amplification (RCA; M. M. Ali, F. Li, Z. Zhang, K. Zhang, D.-K. Kang, J. A. Ankrum, X. C. Le and W. Zhao, Chem. Soc. Rev., 2014, 43, 3324-3341), loop-mediated isothermal amplification (LAMP; Y. Mori, H. Kanda and T. Notomi, J. Infect. Chemother., 2013, 19, 404-411), recombinase polymerase amplification (RPA; J. Li, J. Macdonald and F. von Stetten, Analyst, 2018, 144, 31-67), nucleic acid sequence based amplification (NASBA; A. Borst, J. Verhoef, E. Boel and A. C. Fluit, Clin. Lab., 2002, 48, 487-492), strand displacement amplification (SDA; B. J. Toley, I. Covelli, Y. Belousov, S. Ramachandran, E. Kline, N. Scarr, N. Vermeulen, W. Mahoney, B. R. Lutz and P. Yager, Analyst, 2015, 140, 7540-7549), helicase dependent amplification (HAD; M. Vincent, Y. Xu and H. Kong, EMBO Rep., 2004, 5, 795-800), transcription mediated amplification (TMA; L. Comanor, Am. J. Gastroenterol., 2001, 96, 2968-2972).

### VI. Method for eliminating carryover contamination

According to still another aspect of the present invention, there is provided a method for eliminating carryover contamination in a nucleic acid amplification reaction, comprising the step of incubating a composition for the nucleic acid amplification reaction with a sample suspected of containing the nucleic acid, wherein the composition comprises uracil-DNA glycosylase (UDG) derived from *Photobacterium leiognathi.*

As described above, the UDG comprises the amino acid sequence represented by SEQ ID NO: 2. In one embodiment, the UDG is encoded by the nucleotide sequence represented by SEQ ID NO: 1.

Details of the composition for a nucleic acid amplification reaction and a nucleic acid amplification reaction can be found in Section V above.

In the method of the present invention, incubating a composition for a nucleic acid amplification reaction with a sample suspected of containing a nucleic acid means that the composition for a nucleic acid amplification reaction is mixed with the sample suspected of containing a nucleic acid and the mixture is then placed at an appropriate temperature for an appropriate time.

In one embodiment, the incubating is performed at 10 to 25°C. In certain embodiments, the incubating is performed for a period of 30 minutes or less, such as 1 to 30 minutes, 1 to 20 minutes, 1 to 10 minutes, or 1 to 5 minutes. In certain embodiments, the incubating is performed for a very short period of time of less than 1 minute.

In the method of the present invention, the incubating may occur during the set-up process for a nucleic acid amplification reaction. The set-up process of a nucleic acid amplification reaction refers to a process of mixing a composition for a nucleic acid amplification reaction with a sample in a reaction vessel. The mixture of the composition for nucleic acid amplification reaction and the sample are left for a predetermined time until the nucleic acid amplification reaction is initiated. Being left for a predetermined time after mixing corresponds to the incubating in the method of the present invention.

The UDG of the present invention has an activity of specifically degrading only uracil bases in a DNA substrate, and is thus able to eliminate carryover contamination occurring during a nucleic acid amplification reaction. The carryover contamination refers to contamination that occurs in a nucleic acid extraction process, a nucleic acid amplification reaction process of a sample, or the like for a nucleic acid amplification reaction, and preferably, the carryover contamination may be due to a uracil base that is externally added to a DNA substrate, a uracil base that is naturally deaminated from cytosine and inserted into DNA, or a uracil base that is present in DNA that is replicated after insertion of the uracil base.

Therefore, the method for eliminating carryover contamination in a nucleic acid amplification reaction according to the present invention comprises reacting a uracil-containing substrate and the composition for a nucleic acid amplification reaction comprising the UDG of the present invention in a temperature range (e.g., 10-25°C.) in which the UDG of the present invention exhibits activity, and then performing a nucleic acid amplification reaction according to a procedure known in the art. The UDG of the present invention loses its activity over a temperature of 50°C during a nucleic acid amplification reaction after the enzymatic reaction and thus can prevent degradation of or reduction in amount of products of the nucleic acid amplification reaction.

Thus, the method of the invention comprises the steps of incubating the composition for a nucleic acid amplification reaction comprising the UDG according to the invention with the sample, and inactivating the UDG.

The step of inactivating the UDG may be performed during or before the nucleic acid amplification reaction.

In one embodiment, when the nucleic acid amplification reaction is PCR or real-time PCR, the UDG is inactivated at a temperature of at least 50°C during PCR or real-time PCR.

In another embodiment, where the nucleic acid amplification reaction is an isothermal amplification reaction below 50°C, the UDG is inactivated by incubating it at a temperature of at least 50°C prior to the isothermal amplification reaction.

It was confirmed by the present inventors that as a result of reacting the composition for a nucleic acid amplification reaction comprising the UDG of the present invention with a sample containing contaminated uracil-DNA at 25°C for 5 minutes and performing a PCR reaction, the UDG had no effect on the PCR reaction while eliminating carryover contamination occurring during the PCR reaction (see Examples 5 and 6).

Therefore, it could be confirmed that the UDG enzyme derived from *Photobacterium leiognathi* of the present invention specifically select and eliminate only the uracil bases from the DNA substrate.

### VII. Method for producing UDG

According to another aspect of the present invention, there is provided a method for producing uracil-DNA glycosylase (UDG) derived from *Photobacterium leiognathi,* comprising the steps of:
(a) culturing a host cell transformed to express UDG derived from *Photobacterium leiognathi;*
(b) obtaining the UDG in the form of inclusion bodies from the cultured host cell;
(c) refolding the resulting UDG in the form of inclusion bodies; and
(d) purifying the refolded UDG.

As described above, the UDG comprises the amino acid sequence represented by SEQ ID NO: 2.

In an embodiment, the UDG is encoded by the nucleotide sequence represented by SEQ ID NO: 1.

In an embodiment, the host cell is *Escherichia coli.*

In an embodiment, the host cell is transformed with an expression vector comprising the nucleotide sequence represented by SEQ ID NO: 1.

In an embodiment, the expression vector comprises a T7 promoter.

In an embodiment, the UDG produced by the method does not contain DNA or RNA derived from *E. coli.*

Hereinafter, the steps of the method according to Section VII will be described in detail.

### Step (a): Culturing a transformed host cell

In this step, a host cell transformed to express UDG derived from *Photobacterium leiognathi* is cultured.

Since the expression of the UDG is achieved by the step of culturing the host cell in a culture medium according to an embodiment of the present invention, the description overlapping with those mentioned in relation to the above-described host cell are equally applied.

In one embodiment of the present invention, the culturing of step (a) is performed in the presence of IPTG, when the host cell is transformed with an expression vector having a lac promoter.

In certain embodiments of the present invention, the culturing in step (a) is performed in the presence of IPTG at a concentration of 0.05 mM to 1 mM at 25 to 40°C, but is not limited thereto.

For example, the culture temperature may be 25 to 40°C, 26 to 40°C, 27 to 40°C, 28 to 40°C, 29 to 40°C, 30 to 40°C, 35 to 40°C, 25°C, 26°C, 27°C, 28°C, 29°C, 30°C, 31°C, 32°C., 33°C, 34°C, or 35°C.

In addition, the concentration of IPTG may be 0.05 mM to 1 mM, 0.1 mM to 1 mM, 0.15 mM to 1 mM, 0.2 mM to 1 mM, 0.25 mM to 1 mM, 0.3 mM to 1 mM, 0.4 mM to 1 mM, 0.5 mM to 1 mM, 0.1 mM, 0.15 mM, 0.2 mM, 0.25 mM, 0.3 mM, or the like.

In certain embodiments, the culturing of step (a) is performed by primary culturing at 37°C in a culture medium without IPTG, followed by IPTG-induced culture at 30°C.

### Step (b): Obtaining the UDG in the form of inclusion bodies

In this step, the UDG in the form of inclusion bodies is obtained from the cultured host cell.

As used herein, the inclusion bodies refers to insoluble recombinant proteins produced in the host cell or aggregated recombinant proteins due to incomplete folding. It is generally known that the inclusion bodies occur when the expression level of the protein exceeds 2% of the total cellular proteins. Therefore, the higher the expression level of the recombinant protein, the more likely it is to generate inclusion bodies. It is also likely to occur inclusion bodies if the copy number of the target gene is high or the promoter system is strong.

Conventional UDGs are known to be expressed only in a soluble form upon expression using an expression vector in a host cell. However, it has been found that the UDG of *Photobacterium leiognathi* according to the present invention is not expressed in a soluble form, but in the form of inclusion bodies in *E. coli* as a host cell.

When *E. coli* is used as a host cell for protein expression, the resulting inclusion bodies consist mainly of a recombinant protein of interest. Thus, the development of a method that allow for recovery of recombinant proteins from inclusion bodies in a convenient and efficient manner and induction of appropriate folding of the recombinant proteins can avoid extensive purification processes in producing recombinant proteins from *E coli.*

In one embodiment of the present invention, the UDG in the form of inclusion bodies is obtained by lysing the host cell. The lysis of the host cell may be achieved by a process consisting of physical disruption, chemical disruption, or a combination thereof of the transformed host cell.

The physical disruption of the host cell includes, but is not limited to, disruption by ultrasonication, homogenization, ball milling, and the like, and various physical disruption methods available in the art may be used.

The chemical disruption of the host cell includes, but is not limited to, treatment with a cell lysis agent, and various chemical disruption methods available in the art may be used. As an example, lysozyme may be used for cell lysis.

In one embodiment, the method of the present invention further comprises washing the inclusion bodies obtained after step (b).

The washing may be performed using a buffer comprising urea, Tris-HCl, NaCl, Triton X-100, or a combination thereof.

In a specific embodiments of the present invention, the washing is performed using a buffer containing 1 to 3 M urea, 10 to 30 mM Tris-HCl, 0.1 to 1.0 M NaCl, and 1 to 3% Triton X-100, but is not limited thereto. In a more specific embodiment, the washing is performed using a buffer comprising 2 M urea, 20 mM Tris-HCl, 0.5 M NaCl, and 2% Triton X-100.

The buffer used for washing has the effect of washing the inclusion bodies by eliminating membrane fragments, whereby RNA and DNA derived from host cells are eliminated.

In one embodiment, the method of the present invention further comprises solubilizing the resulting inclusion bodies after step (b).

The solubilizing may be performed using one or more buffers.

In one embodiment, the solubilizing is performed using a buffer comprising Tris-HCl, NaCl, imidazole, guanidine hydrochloride, 2-mercaptoethanol, or a combination thereof.

In a specific embodiment of the present invention, the solubilizing is performed using a buffer containing 10 to 30 mM Tris-HCl, 0.1 to 1 M NaCl, 1 to 10 mM imidazole, 5 to 7 M guanidine hydrochloride and 0.1 to 10 mM 2-mercaptoethanol, but is not limited thereto. In a more specific embodiment, the solubilization is performed using a buffer comprising 20 mM Tris-HCl, 0.5 M NaCl, 5 mM imidazole, 6 M guanidine hydrochloride and 0.1 to 10 mM 2-mercaptoethanol.

### Step (c): Refolding of the UDG in the form of inclusion bodies

In this step, the resulting UDG in the form of inclusion bodies is refolded.

In an embodiment, the refolding of step (c) is performed on a column in column chromatography.

Specifically, the step comprises loading the solubilized UDG onto the column chromatography to bind it to the column, and then refolding the UDG while bound to the column.

In an embodiment, the column chromatography comprises size exclusion chromatography, ion exchange chromatography, affinity chromatography, hydrophobic interaction chromatography, or a combination thereof.

In certain embodiments, the column chromatography is affinity chromatography, e.g., chromatography having an affinity column capable of binding to the UDG comprising a His tag expressed according to the methods of the present invention.

The columns as used herein can be equilibrated using an equilibration buffer. In an embodiment, the equilibration buffer comprises Tris-HCl, NaCl, imidazole, guanidine hydrochloride, 2-mercaptoethanol, or a combination thereof. In another embodiment, the equilibration buffer comprises 10 to 30 mM Tris-HCl, 0.1-1M NaCl, 1-10 mM imidazole, 5-7 M guanidine hydrochloride, and 0.1-10 mM 2-mercaptoethanol. In another embodiment, the equilibration buffer is the same as the buffer for solubilizing UDG.

In this step, the solubilized UDG is loaded onto a pre-equilibrated column chromatography, e.g., a His tag column chromatography, thereby binding it to the column.

In this step, the solubilized UDG bound to the column is in a denatured state.

In one embodiment, this step comprises washing the column to which the UDG is bound.

The washing of the column may be performed using a column wash buffer.

In one embodiment of the invention, the column wash buffer comprises urea. In a specific embodiment, the column wash buffer comprises 10-30 mM Tris-HCl (pH 8.0), 0.1-1.0 M NaCl, 10-30 mM imidazole, 5-7 M urea, and 0.1-10 mM 2-mercaptoethanol. In a more specific embodiment, the column wash buffer comprises 20 mM Tris-HCl (pH 8.0), 0.5 M NaCl, 20 mM imidazole, 6 M urea, and 1 mM 2-mercaptoethanol.

In this step, refolding of the solubilized UDG bound to the column is induced to restore the activity and function of the UDG. The step may be termed as on column refolding.

In an embodiment of the present invention, the on-column refolding may be performed by passing a buffer suitable for refolding through a column to with the solubilized UDG is bound.

Suitable buffers for refolding comprises Tris-HCl, NaCl, imidazole, 2-mercaptoethanol, or combinations thereof.

In an embodiment of the present invention, the buffer may include additional additives such as amino acids, sugars, polyalcohols, non-detergent zwitterions, and combinations thereof.

The amino acids include, without limitation, glycine, arginine, proline, or a combination thereof.

The sugars include, without limitation, sucrose, sorbitol, or a combination thereof.

The polyalcohols include, without limitation, polyethylene glycol, glycerol, or a combination thereof.

The non-detergent zwitterions include, without limitation, sulfobetaine, substituted pyrimidine and pyrrole, and acid substituted aminocyclohexane.

In an embodiment, the refolding is performed by applying a urea gradient from high concentration to low concentration on the column in column chromatography. Specifically, the refolding comprises passing buffer solutions through the column, wherein a buffer solution containing a higher concentration of urea is exchanged for another buffer solution containing a lower concentration of urea. In certain embodiments, the refolding is performed using a urea gradient from a buffer containing 5-7 M urea to a buffer containing no urea.

### Step (d): Purifying the refolded UDG

In the step, the refolded UDG is purified.

Prior to the purification, the refolded UDG can be eluted and separated from the column.

The elution of the UDG can be performed by a variety of methods known in the art as long as it does not affect the activity or function of the UDG and is suitable for the principle of the column used for refolding.

In the method of the present invention, the purification of the UDG may be performed by various methods known in the art, for example, the UDG eluted from the column may be purified by performing additional column chromatography, for example, Q column chromatography, but is not limited thereto.

Conventional methods for expressing and purifying a target protein in *E. coli* comprise purifying the target protein from a supernatant secreted from *E. coli,* and the target protein purified by this method comprises DNA or RNA from *E. coli.* That is, since UDGs as expressed and purified in *E. coli* by conventional methods comprise DNA or RNA from *E. coli,* such UDGs cannot be used to detect *E. coli* (even if a sample does not contain *E. coli,* it is likely to erroneously determine the presence of *E. coli* in the sample). Therefore, there is a need to reduce contamination by DNA and RNA derived from *E. coli* in the expression and purification of UDGs.

In contrast, the method of the present invention can effectively reduce the possibility that UDG includes DNA and RNA from *E. coli,* i.e., contamination due to DNA and RNA from *E. coli,* by expressing and purifying the UDG in the form of inclusion bodies in *E. coli.*

Furthermore, the use of the method of the present invention, i.e. by solubilizing the UDG in the form of inclusion bodies and binding the solubilized UDG to a column, followed by on column refolding, can effectively reduce contamination due to DNA and RNA derived from *E. coli,* and produce the UDG in large quantities.

The novel uracil-DNA glycosylase (UDG) of the present invention is an enzyme isolated from *Photobacterium leiognathi,* which is a psychrophilic bacterium, and is characterized by having an activity of degrading uracil bases from uracil-containing DNA substrate and being easily denatured at low temperatures. Accordingly, the UDG of the present invention can be effectively used to eliminate carryover contamination occurring during the nucleic acid amplification reaction, resulting in increased accuracy (elimination of false positives), purity and amplification efficiency in nucleic acid amplification reactions.

### BRIEF DESCRIPTION OF THE DRAWINGS

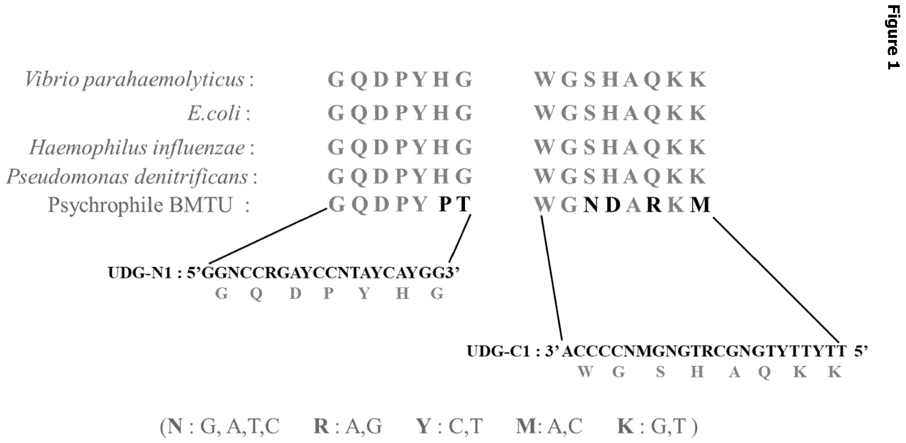

FIG. 1 shows a conserved region between the amino acid sequences of UDGs of *Vibrio parahaemolyticus* (*Vpa*), *Escherichia coli, Haemophilus influenzae* (*Hin*), *Pseudomonas denitrificans* (*Pde*), and BMTU3346, a psychrophilic marine bacterium, as well as sequences of primers for PCR prepared based on the conserved region. For the primer sequences in the figure, N represents G, A, T or C; R represents A or G; Y represents C or T; M represents A or C; and K represents G or T.

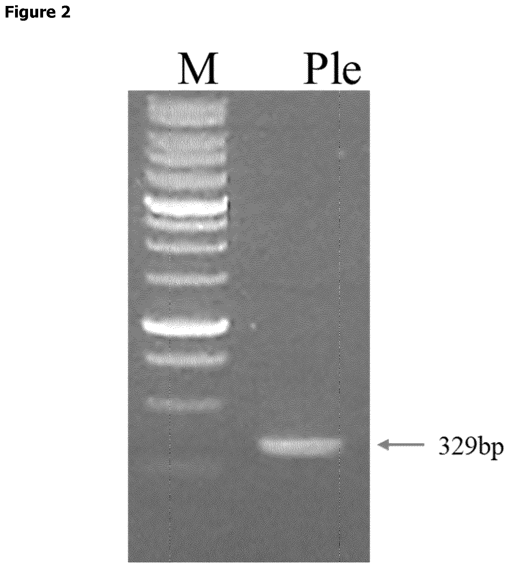

FIG. 2 shows agarose gel electrophoresis results for amplification products of PCR using the genomic DNA of *Photobacterium leiognathi* as a template and primers of SEQ ID NOs: 3 and 4. In the figure, the 1^{st} lane as indicated by M represents a 1 kb ladder marker DNA, and the 2^{nd} lane as indicated by Ple represents the PCR product.

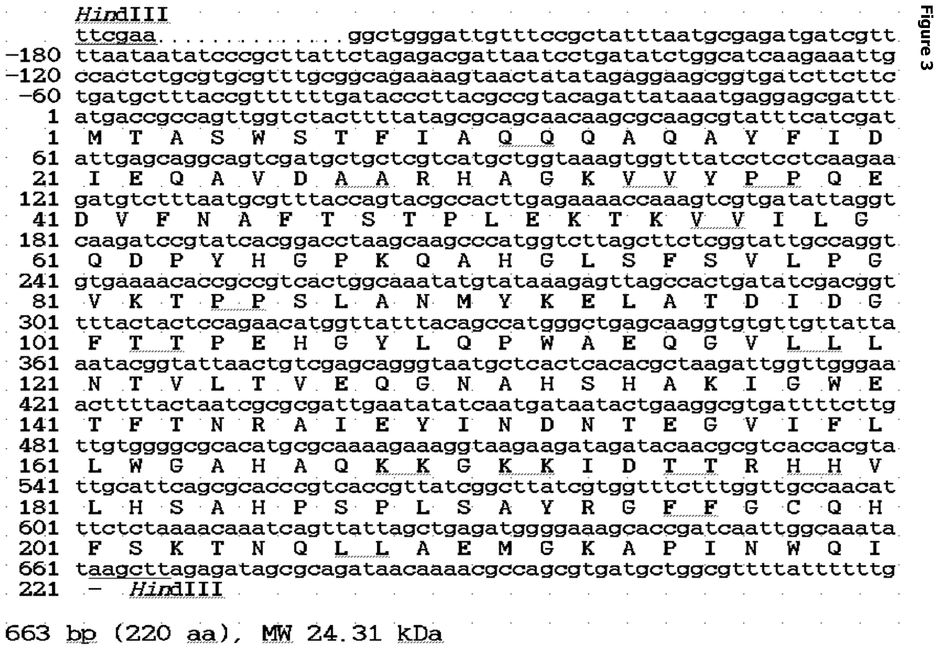

FIG. 3 shows the nucleotide sequence, amino acid sequence, and molecular weight of the entire gene of the UDG of *Photobacterium leiognathi.*

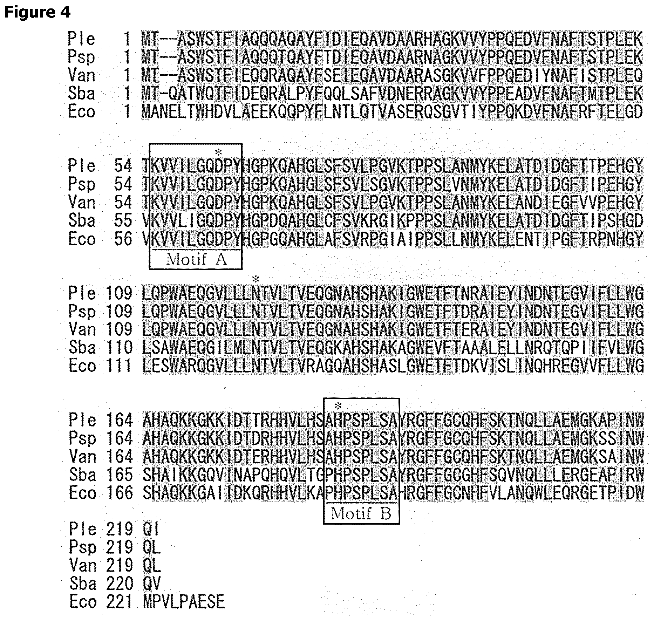

FIG. 4 shows the comparison between amino acid sequences of UDG genes of *Photobacterium leiognathi* strain of the present invention, *Photobacterium sp.* SKA34 (Psp), *Vibrio angustum* S14 (Van), *Shewanella baltica* OS195 (Sba), and *E. coli* (Eco). In the figure, the amino acids known to be involved in the activity of UDG are indicated by an asterisk, and the regions corresponding to the known motifs A and B are indicated by boxes.

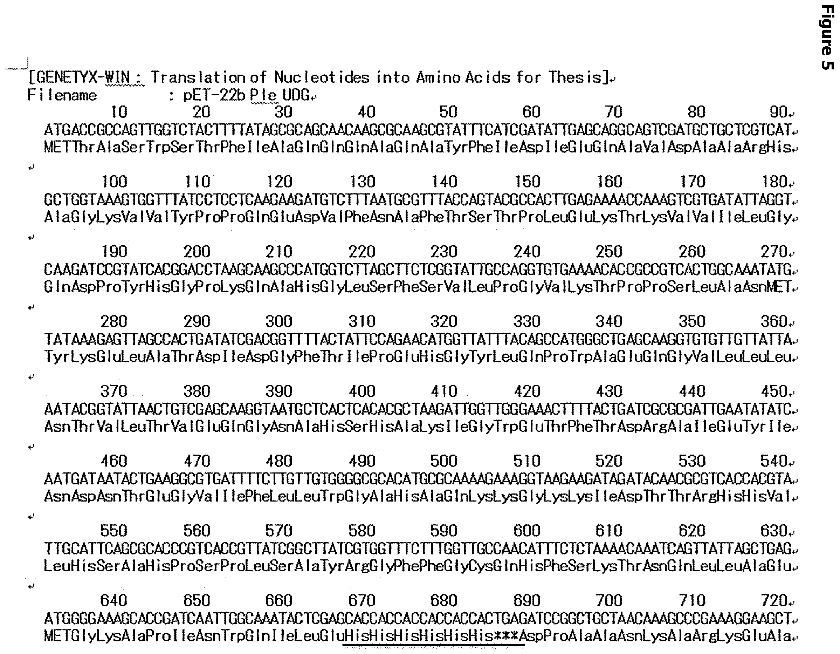

FIG. 5 shows the sequencing result for the UDG gene in a pET-22b vector (pET-22b Ple UDG) comprising the UDG gene according to the present invention.

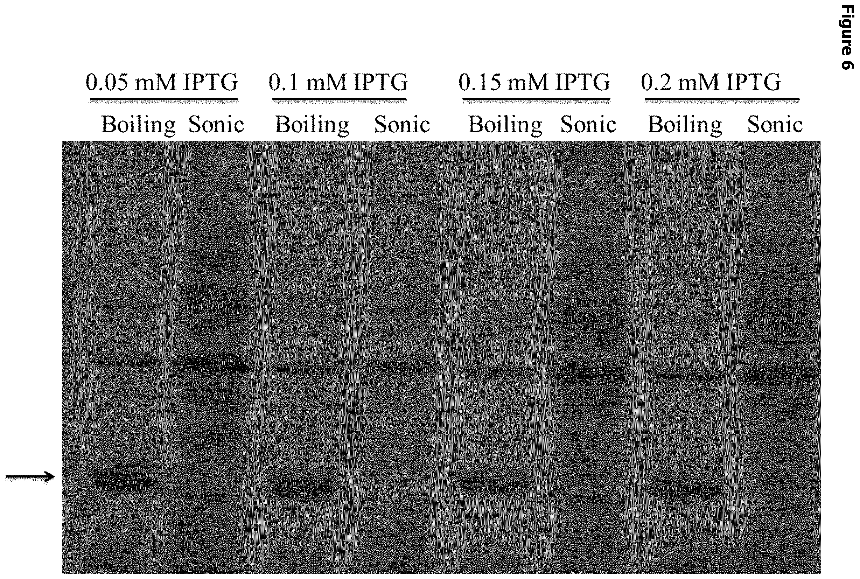

FIG. 6 shows the SDS-PAGE results for the experimental group 1 in which boiled cell pellets were loaded and the experimental group 2 in which sonicated supernatants were loaded, both after *E. coli* transformed with the recombinant UDG (Ple UDG) of the present invention was cultured in a medium containing IPTG at different concentrations. In FIG. 6, the 1^{st} and 2^{nd} lanes show the results for experimental group 1 (denoted by Boiling) in which boiled cell pellets were loaded and experimental group 2 (denoted by Sonic) in which sonicated supernatants were loaded, both after the expression was induced with 0.05 mM IPTG; the 3^{rd} and 4^{th} lanes show the results of Experimental Group 1 and Experimental Group 2 in which the expression was induced with 0.1 mM IPTG; the 5^{th} and 6^{th} lanes with 0.15 mM IPTG, and the 7^{th} and 8^{th} lanes with 0.2 mM IPTG, respectively. In each lane of FIG. 6, a band corresponding to the molecular weight of the UDG of the present invention is also indicated by an arrow.

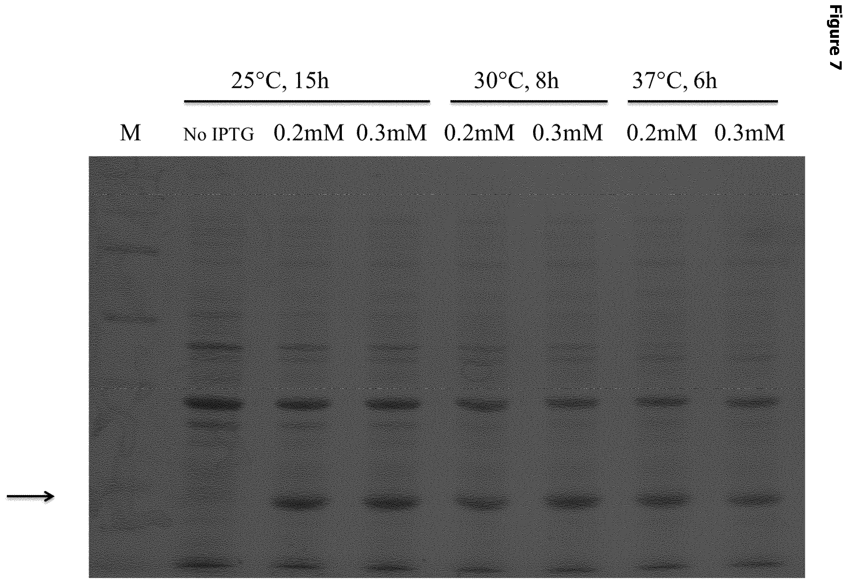

FIG. 7 shows the SDS-PAGE results for the recombinant UDGs (Ple UDG) of the present invention, which expressed in *E. coli* by varying the concentration of IPTG and culture temperature and time. In FIG. 7, the 1^{st} lane represents a molecular weight marker, the 2^{nd} to 4^{th} lanes represent the culture at 25°C for 15 hours with varying concentrations of IPTG, the 5^{th} and 6^{th} lanes represent the culture at 30°C for 8 hours with varying concentrations of IPTG, and the 7^{th} and 8^{th} lanes represent the culture at 37°C for 6 hours with varying concentrations of IPTG. In each lane of FIG. 7, a band corresponding to the molecular weight of the UDG of the present invention is also indicated by an arrow.

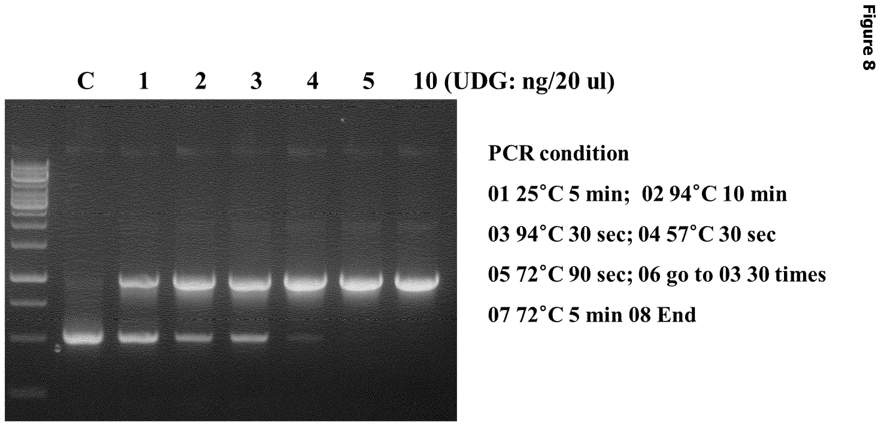

FIG. 8 shows agarose gel electrophoresis results for amplification products of PCR after artificially contaminated uracil-DNA substrates are reacted with a PCR reagent containing various concentrations of the *Ple* UDG of the present invention at 25°C. In FIG. 8, the 1^{st} lane represents a marker DNA, and the 2^{nd} to 8^{th} lanes represent the addition of 0, 1, 2, 3, 4, 5, and 10 ng/20 µl of the *Ple* UDG of the present invention, respectively.

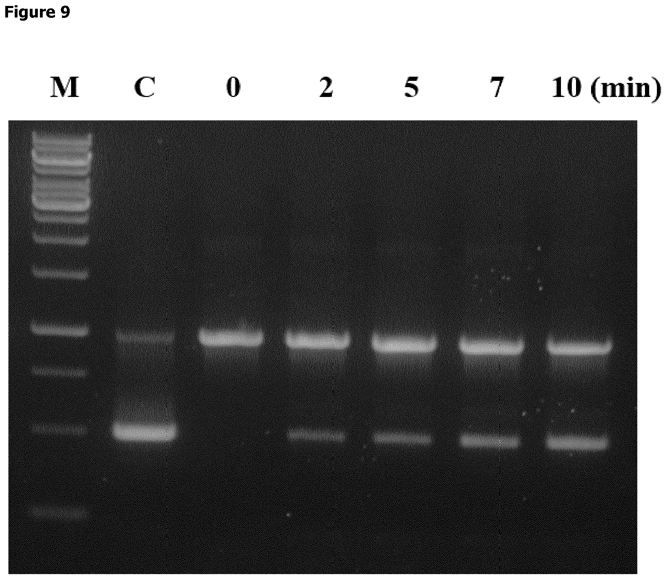

FIG. 9 shows agarose gel electrophoresis results for amplification products of PCR after the *Ple* UDG of the present invention is treated for various times at 50°C and added to contaminated uracil-DNA substrates, followed by reaction at 25°C. In FIG. 9, the 1^{st} lane represents a marker DNA, the 2^{nd} lane represents a PCR mixture not containing the UDG of the present invention, and the 3^{rd} to 7^{th} lanes represent a PCR mixture containing the UDG of the present invention, which is treated at 50°C for 0, 2, 5, 7 or 10 minutes.

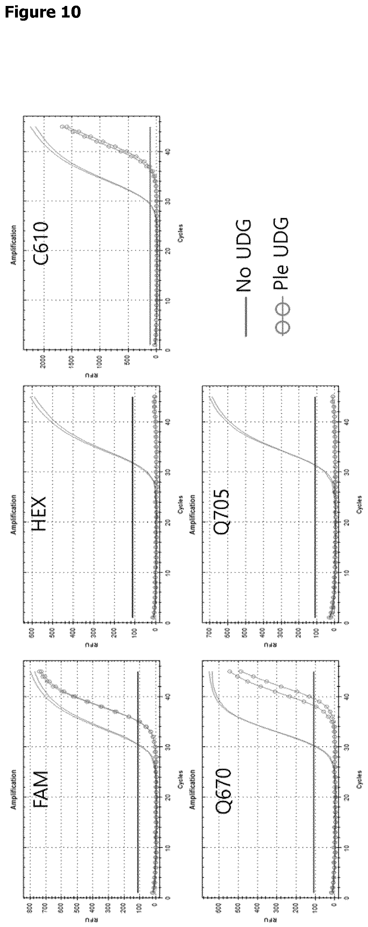

FIG. 10 shows the results of PCR reactions for 5 ul of a positive control (PC) of Novaplex^{™} Urinary Tract Infection (UTI) Panel 1 (cat. No. R-SD10228W, Seegene Inc.), which is a multiplex real-time PCR product capable of detecting a plurality of targets in a single tube, and oligonucleotides capable of amplifying the PC, with or without the UDG of the present invention.

### DETAILED DESCRIPTION OF THIS INVETNION

The present invention will now be described in further detail by examples. It would be obvious to those skilled in the art that these examples are intended to be more concretely illustrative and the scope of the present invention as set forth in the appended claims is not limited to or by the examples.

### Examples

Throughout this specification, "%" as used to indicate the concentration of a particular substance refers to (weight/weight)% for solid/solid, (weight/volume)% for solid/liquid, and (volume/volume)% for liquid/liquid, unless otherwise stated.

### Example 1: Cloning of the uracil-DNA glycosylase (UDG) gene

In order to search for a novel uracil-DNA glycosylase (UDG) gene having an activity at low temperatures, the genomic DNA of *Photobacterium leiognathi* (Accession Number ATCC 33980), a psychrophilic strain, which was received from American Type Culture Collection (ATCC) was used as a template. The 16s rRNA of *Photobacterium leiognathi* exhibited high homology to the 16s rRNA of *Photobacterium sp.*

PCR was performed using primers of SEQ ID NOs: 3 and 4, which were prepared based on a conserved region among previously reported amino acid sequences of UDGs of *Vibrio parahaemolyticus* (*Vpa*), *Escherichia coli, Haemophilus influenzae* (*Hin*), *Pseudomonas denitrificans*(*Pde*), and BMTU3346, a psychrophilic marine bacterium (see FIG. 1):
Forward primer UDG-N1: 5'-GGNCCRGAYCCNTAYCAYGG-3' (SEQ ID NO: 3)
Reverse primer UDG-C1: 5'-TTYTTYTGNGCRTGNGMNCCCCA-3' (SEQ ID NO: 4)
(wherein N represents G, A, T or C; R represents A or G; Y represents C or T; M represents A or C; and K represents G or T) (see FIG. 1).

The PCR condition was as follows: 95°C for 3 minutes, 5 cycles consisting of 94°C for 1 minute, 53°C for 1 minute, and 68°C for 1 minute, and then 25 cycles consisting of 94°C for 1 minute, 57°C for 1 minute, and 70°C for 1 minute, and then extension at 72°C for 10 minutes. The amplification product was electrophoresed on an agarose gel.

The results are shown in FIG. 2. In FIG. 2, the 1^{st} lane as indicated by M represents a 1 kb ladder marker DNA, and the 2^{nd} lane as indicated by Ple represents the PCR product. As shown in FIG. 2, it was found that a DNA fragment of about 329 bp was amplified.

The PCR amplification product was then purified from the agarose gel using the QIAquick Gel Extraction kit (QIAGEN) and cloned into pGEM-T Easy vector system I (Promega) according to the manufacturer's protocol. The cloned DNA was sequenced by Macrogen Co., Ltd.

Then, the nucleotide sequence was translated into amino acid sequence, which was compared with amino acid sequences of UDGs from other known species, i.e., *Vibrio parahaemolyticus, Vibrio fischeri,* and *Vibrio vulnificus* strains.

As a result, it was found that the amino acid sequence of the fragment of the UDG gene from the *Photobacterium leiognathi* strain of the present invention had 80% identity to UDG of *Vibrio parahaemolyticus,* 83% identity to UDG of *Vibrio fischeri,* and 78% identity to UDG of *Vibrio vulnificus.* Therefore, it was confirmed that the cloned 329 bp DNA fragment was part of the UDG gene of the *Photobacterium leiognathi* strain.

### Example 2: Cloning of entire UDG gene

The entire sequence of the UDG of *Photobacterium leiognathiwas* cloned by an inverse PCR method from the partial sequence (329 bp) of the UDG of *Photobacterium leiognathi* obtained in Example 1 above (see Ogasawara, N., et al., DNA Res, 1, 1-14, 1994). Inverse PCR is a method of amplifying an unknown nucleotide sequence flanked by a known nucleotide sequence to determine the unknown nucleotide sequence.

First, the genomic DNA of the *Photobacterium leiognathi* strain was digested with the restriction enzyme *Hin*dIII, and then the fragments were purified by phenol treatment. About 1 µg of the purified DNA fragment was added with T₄ DNA ligase and a reaction buffer to make the total volume 20 ul, followed by self-ligation overnight at 16°C. Thereafter, in order to perform inverse PCR using the self-ligated DNA products as a template, two internal primers (SEQ ID NOs: 5 and 6) were prepared based on the nucleotide sequence of the conserved gene of the UDG corresponding to about 329 bp, which was identified from the *Photobacterium leiognathi* strain of Example 1.

### Internal primer sequences

Forward primer PleN1: 5'-AAGACCATGGGCTTGCTTAGG-3' (SEQ ID NO: 5)
Reverse primer PleC1: 5'-TCAATGATAATACTGAAGGCGTG-3' (SEQ ID NO: 6)

The inverse PCR was performed using the two internal primers and the self-ligated DNA products as a template. The PCR condition was as follows: 95°C for 3 minutes, 30 cycles consisting of 94°C for 30 seconds, 55°C for 1 minute, and 72°C for 2 minutes, and then extension at 72°C for 10 minutes. The PCR amplification product was electrophoresed on a 0.8% agarose gel, confirming that a DNA fragment of about 0.7 kb was amplified. The PCR amplification product was sequenced by Macrogen Co., Ltd. The DNA nucleotide sequence was analyzed using the DNASTAR program to determine the nucleotide sequence of the entire UDG gene.

As a result, the entire nucleotide sequence of the UDG gene isolated from the *Photobacterium leiognathi* strain was 663 bp in total, from the start codon (ATG) to the stop codon (TAA) (see SEQ ID NO: 1), and was 220 amino acids in total (see SEQ ID NO: 2). Based on the amino acid sequence, it could be estimated that the molecular weight of the UDG of the present invention was about 24.31 kDa (FIG. 3).

In addition, the amino acid sequence of the UDG gene isolated from the *Photobacterium leiognathi* strain of the present invention was compared with amino acid sequences of UDG genes of *Photobacterium sp.* SKA34 (Psp), *Vibrio angustum* S14 (Van), *Shewanella baltica* OS195 (Sba), and *E. coli,* using the NCBI program (NCBI BLAST program). The results are shown in FIG. 4.

As shown in FIG. 4, the UDG of the present invention showed 95% sequence homology with the UDG of *Photobacterium sp.* SKA34 (Psp), 90% homology with the UDG of *Vibrio angustum* S14 (Van), 68.6% homology with the UDG of *Shewanella baltica* OS195 (Sba), and 61.8% sequence homology with the UDG of *E. coli* (Eco).

In addition, it is known that the 62^{nd} amino acid D (Asp), the 121^{st} amino acid N (Asn), and the 185^{th} amino acid H (His) are important amino acids involved in the activity of UDG (see Sartori, A. A. et al., EMBO J 21, 3182-3191, 2002), and as shown in FIG. 4, the three amino acids (indicated by asterisks in the figure) in the UDG of the present invention was well conserved. In particular, the UDG of the present invention was found to have a motif A comprising the 62^{nd} amino acid D (ASP) and motif B comprising the 185^{th} amino acid H (His), both of which are well conserved.

### Example 3: Preparation of a construct expressing the recombinant UDG and transformation using the same

The genomic DNA of *Photobacterium leiognathiwas* amplified by PCR using primers of SEQ ID NOs: 7 and 8 into which either *Nde*I or *Xho*I was artificially inserted.
PleNde primer: 5-'NNNNNCATATGACCGCCAGTTGGTCTAC-3' (SEQ ID NO: 7)
PleXho primer: 5'-NNNNNCTCGAGTATTTGCCAATTGATCGGTGCTTTC-3' (SEQ ID NO: 8)
wherein the underlined "CATATG" indicates the *Nde*I restriction site and the underlined "CTCGAG" indicates the *Xho*I restriction site.

The PCR amplification product was digested with *Nde*I and *XhoI,* and then electrophoresed on a 1.2% agarose gel to isolate the UDG gene (excluding stop codons) of 663 bp. The isolated DNA fragment was digested with the same *Nde*I and *XhoI* restriction enzymes and purified.

Meanwhile, an expression vector pET-22b (Novagen, USA) having a T7 promoter was digested with *Nde*I and *XhoI,* and the digested product was ligated with the UDG gene of 663 bp, which was treated with the same restriction enzymes, using T₄ DNA ligase overnight at 16°C, thereby constructing an expression vector pET-22b *Ple* UDG.

The constructed pET-22b *Ple* UDG was transformed into *E. coli* BL21(DE3) by Hanahan method (see Hanahan, D. et al., Gene 10, 63-67, 1983). The transformed strain was then plated on LB plates supplemented with 100 µg/mL of ampicillin and cultured overnight at 37°C. The transformants grown on the LB plate were cultured again, and then the plasmid pET-22b *Ple* UDG was isolated. Then, the plasmid was digested with restriction enzymes *Nde*I and *XhoI* and electrophoresed on a 1.2% agarose gel.

Thereafter, the UDG gene was separated from the agarose gel, and sequenced. The results are shown in FIG. 5.

As shown in FIG. 5, the pET-22b *Ple* UDG constructed in this Example was found to contain a 663 bp fragment of the UDG gene followed by six histidine tags.

### Example 4: Expression and purification of UDG

In order to express the UDG enzyme of the present invention as a fusion protein from the recombinant strain (pET-22b *Ple* UDG/BL21(DE3)) transformed into *E. coli* BL21 (DE3) by the method of Example 3, and to purify only the UDG of the present invention from the expressed fusion protein, the following experiment was performed.

### 4-1. Determination of an optimal IPTG concentration and an expression site of Ple UDG

First, in order to confirm the optimal IPTG concentration for the expression of the *Ple* UDG and the expression site of the *Ple* UDG using the recombinant strain, the following experiment was performed.

The recombinant strain prepared in Example 3 was inoculated into an LB broth medium supplemented with 100 µg/mL of ampicillin and cultured overnight at 37°C, and then 300 µl of the culture was inoculated into 30 ml of the same medium and cultured at 37°C. When an OD₆₀₀ of about 0.65 was reached, the culture solution was cooled on ice, and then incubated at 16°C for about 20 hours with varying concentrations of IPTG (0.05, 0.1, 0.15, 0.2 mM IPTG).

The culture solution was diluted 10 times and the dilute was measured for OD₆₀₀ value. Thereafter, a volume corresponding to an OD₆₀₀ of 1.0 was centrifuged at 6,000 rpm for 15 minutes, the recovered cell pellet was added with 80 µl of distilled water and 20 µl of 5x SDS buffer, the mixture was boiled for 5 minutes, and 20 µl of the mixture was loaded onto SDS-PAGE (Experimental Group 1). Meanwhile, 5 ml of the culture solution was sonicated, centrifuged, and 32 µl of the supernatant was loaded onto SDS-PAGE (Experimental Group 2). Experimental Group 1, in which the cell pellets were boiled, contained only UDG in the form of inclusion bodies expressed in cells, whereas Experimental Group 2, in which the supernatant was taken after the sonication, contained only UDG secreted outside the cells.

The SDS-PAGE results are shown in FIG. 6.

In FIG. 6, the 1^{st} and 2^{nd} lanes show the results for experimental group 1 (denoted by Boiling) in which boiled cell pellets were loaded and experimental group 2 (denoted by Sonic) in which sonicated supernatants were loaded, both after the expression was induced with 0.05 mM IPTG; the 3^{rd} and 4^{th} lanes show the results of Experimental Group 1 and Experimental Group 2 in which the expression was induced with 0.1 mM IPTG; the 5^{th} and 6^{th} lanes with 0.15 mM IPTG, and the 7^{th} and 8^{th} lanes with 0.2 mM IPTG, respectively. In each lane of FIG. 6, a band corresponding to the molecular weight of the UDG of the present invention is also indicated by an arrow.

As shown in FIG. 6, it was confirmed that the *Ple* UDG of the present invention corresponding to 24.31 kDa was expressed in the 1^{st}, 3^{rd}, 5^{th}, and 7^{th} lanes. That is, when the recombinant strain (pET-22b *Ple* UDG/BL21 (DE3)) was cultured in a medium containing four different concentrations of IPTG, the *Ple* UDG was observed in Experimental Group 1 in which the cell pellet was boiled, but not in Experimental Group 2 in which the supernatant was taken after sonication.

From the above results, it could be seen that the *Ple* UDG of the present invention was expressed as inclusion bodies in the cell without being secreted out of the cell in *E. coli.*

### 4-2. Determination of expression temperature

In order to determine the optimal culture temperature for the recombinant strain for expression of the *Ple* UDG, the recombinant strain prepared in Example 3 was inoculated into an LB broth medium supplemented with 100 µg/mL of ampicillin and cultured overnight at 37°C, and then 30 µl was inoculated into 3 ml of the same medium and cultured again at 37°C. After 2 hours and 45 minutes of culture, different concentrations (0 mM, 0.2 mM, 0.3 mM) of IPTG were added and incubated for 15 hours at 25°C, 8 hours at 30°C, or 6 hours at 37°C, respectively. The culture solution was diluted 10 times to measure the OD₆₀₀ value, and the cells were recovered by centrifugation at 6,000 rpm for 15 minutes. The cells were mixed with 80 µl of distilled water and 20 µl of 5x SDS buffer, the mixture was boiled and centrifuged, and 20 µl was loaded onto SDS-PAGE. The results are shown in FIG. 7.. In FIG. 7, the 1^{st} lane represents a molecular weight marker, the 2^{nd} to 4^{th} lanes represent the culture at 25°C for 15 hours with varying concentrations of IPTG, the 5^{th} and 6^{th} lanes represent the culture at 30°C for 8 hours with varying concentrations of IPTG, and the 7^{th} and 8^{th} lanes represent the culture at 37°C for 6 hours with varying concentrations of IPTG.

As shown in FIG. 7, it was found that the UDG of the present invention was well expressed when cultured with addition of IPTG at the three culture temperatures.

### 4-3. Culture for the expression of Ple UDG

The recombinant strain prepared in Example 3 was inoculated into an LB broth medium supplemented with 100 µg/mL of ampicillin and cultured overnight at 37°C, and 2.5 ml of the culture was inoculated into 250 ml of the same medium and then cultured at 37°C. After 2 hours and 30 minutes of culture (OD₆₀₀ measurement of about 0.63), 0.5 ml of 100 mM IPTG was added (0.2 mM of final IPTG concentration), and induced culture was performed at 30°C for 11 hours. After incubation, the culture was diluted 10-fold, and the final OD₆₀₀ (0.62) was measured.

The cells were recovered by centrifugating the culture at 6,000 rpm for 15 minutes and discarding the supernatant. The resulting cells had a weight of about 1.78 g.

### 4-4. Cell lysis

About 1.78 g of the recovered cells were suspended in 25 ml of buffer containing 1 mM PMSF (Phenylmethylsulfonyl fluoride, protease inhibitor), 20 mM Tris-HCl (pH 8.0), and 0.5 mM NaCl. After freezing at -70°C and thawing twice, 1 mg of lysozyme was added per 1 g of the cells, sonication was applied three times for 1 minute and 30 seconds (6 sec on/9 sec off, 25%) to eliminate viscosity by DNA cleavage, and passed several times using a 20G needle in order to eliminate viscosity. Finally, centrifugation was performed at 6000 rpm for 15 minutes to recover the inclusion body.

### 4-5. On-column refolding of Ple UDG inclusion body

In order to eliminate membrane proteins from the recovered inclusion bodies, the inclusion bodies were suspended in 10 ml of an inclusion body washing buffer (2 M urea, 20 mM Tris-HCl (pH 8.0), 0.5 M NaCl, 2% Triton X-100), and the pellets were dispersed three times by sonication. The dispersion was centrifuged, and the precipitate was then mixed with the buffer to disperse the pellets again in the same manner (performed three times in total). Then, the dispersion was centrifuged again, the precipitate was mixed with 20 mM of a Tris-HCl (pH 8.0) buffer, and the pellets were sonicated to disperse it, which was repeated twice. This process allows elimination of membrane proteins, RNA and DNA components derived from *E. coli* used for *Ple* UDG expression.

Then, a His-tag column (5 ml) was prepared by equilibrating it with an inclusion body-His-tag column binding buffer (20 mM Tris-HCl (pH 8.0), 0.5 M NaCl, 5 mM imidazole, 6 M guanidine hydrochloride, and 1 mM 2-mercaptoethanol).

Then, the precipitate was dissolved in 15 ml of the inclusion body-His-tag column binding buffer (20 mM Tris-HCl (pH 8.0), 0.5 M NaCl, 5 mM imidazole, 6 M guanidine hydrochloride, 1 mM 2-mercaptoethanol), the suspension was centrifuged, and the supernatant was loaded onto the His-tag column. Since the His-tag column binding buffer serves to solubilize the inclusion body contained in the precipitate, the supernatant will contain the *Ple* UDG present in the inclusion body.

Next, an inclusion body-His-tag column washing buffer (20 mM Tris-HCl (pH 8.0), 0.5 M NaCl, 20 mM imidazole, 6 M urea, 1 mM 2-mercaptoethanol) was passed through the His-tag column. Then, a His-tag column washing buffer containing urea under a urea gradient from 6 M to 0 M and an on-column refolding buffer containing no urea (20 mM Tris-HCl, pH 8.0, 0.5 M NaCl, 20 mM imidazole, 1 mM 2-mercaptoethanol pH 8.0) were passed through the His-tag column. The urea gradient from 6 M concentration to 0 M concentration allows for refolding of the denatured *Ple* UDG on the column to convert it into the active *Ple* UDG. Refolding was performed at a flow rate of 0.5 ml per minute (volume 120 ml, gradient 100 ml) for about 4 hours, and then additionally performed with 20 ml of the on-column refolding buffer containing no urea.

Then, His-tag column chromatography was performed under an imidazole concentration gradient using a binding and washing buffer (20 mM Tris-HCl (pH 7.0-8.0), 300 mM NaCl, 30 mM imidazole and 1 mM 2-mercaptoethanol) and an elution buffer (20 mM Tris-HCl (pH 7.0-8.0), 300 mM NaCl, 300 mM imidazole and 1 mM 2-mercaptoethanol) to obtain *Ple* UDG eluates.

About 25 ml of the *Ple* UDG eluates (pH 8.0) was diluted by mixing it with 100 ml of 20 mM Tris-HCl (pH 8.0), followed by loading onto a Q column and purification (0-0.5 M concentration gradient of NaCl, flow rate 5 ml/min, fraction: 5 ml, set length: 100 ml, set target: 50%). The eluted *Ple* UDG was dialyzed in a storage buffer (20 mM Tris-HCl, pH 7.4, 50 mM KCl, and 50% glycerol) and stored at -20°C.

The buffers used in the on-column refolding of the *Ple* UDG inclusion bodies of the present invention are shown in Tables 1 to 3 below.

**<Table 1>**

| **Inclusion body washing buffer** | **Amount** |
|---|---|
| 2 M urea (MW 60.06) | 60.6 g |
| 20 mM Tris-HCl (pH 8.0) (1M) | 10 ml |
| 0.5 M NaCl (MW 58.44) | 14.61 g |
| 2% Triton X-100 | 10 ml |

| **Inclusion body-His-tag column binding buffer (500 ml) (Inlet A)** | **Amount** |
|---|---|
| 20 mM Tris-HCl (pH 8.0) (1M) | 10 ml |
| 0.5 M NaCl (MW 58.44) | 14.61 g |
| 5 mM imidazole (MW 68.08) | 170.2 mg |
| 6 M guanidine hydrochloride (MW 95.53) | 286.59 g |
| 1 mM 2-mercaptoethanol (14.26M) | 35.063 ul |

| **Inclusion body-His-tag column washing buffer (500 ml)** | **Amount** |
|---|---|
| 20 mM Tris-HCl (pH 8.0) (1M) | 10 ml |
| 0.5 M NaCl (MW 58.44) | 14.61 g |
| 20 mM imidazole (MW 68.08) | 680.8 mg |
| 6 M urea (MW 60.06) | 180.18 g |
| 1 mM 2-mercaptoethanol (14.26M) | 35.063 ul |

**<Table 2>**

| **On column refolding buffer (500 ml) (Inlet B)** | **Amount** |
|---|---|
| 20 mM Tris-HCl (pH 8.0) (1M) | 10 ml |
| 0.5 M NaCl (MW 58.44) | 14.61 g |
| 20 mM imidazole (MW 68.08) | 680.8 mg |
| 1 mM 2-mercaptoethanol (14.26M) | 35.063 ul |

**<Table 3>**

| **Binding and washing buffer: 20 mM Tris-HCl pH 7-8, 300 mM NaCl, 30 mM imidazole, 1 mM 2-mercaptoethanol (1 liter) (Inlet A)** | **Amount** |
|---|---|
| 0.5 M Tris-HCl | 40 ml |
| 0.3 M NaCl (MW 58.44) | 17.532 g |
| 30 mM imidazole (MW 68.08) | 2.042 g |
| 2 mM 2-mercaptoethanol (14.26M) | 70.126 ul |

| **Elution buffer: 20 mM Tris-HCl (pH 7-8), 300 mM NaCl, 300 mM imidazole, 1 mM 2-mercaptoethanol (Inlet B)** | **Amount** |
|---|---|
| 0.5 M Tris-HCl | 40 ml |
| 0.3 M NaCl (MW 58.44) | 17.532 g |
| 300 mM imidazole (MW 68.08) | 20.424 g |
| 2 mM 2-mercaptoethanol (14.26M) | 70.126 ul |

### Example 5: Effect of UDG on preventing contamination in PCR reaction

In order to investigate whether the UDG of the present invention has an effect of preventing carryover contamination in PCR reactions, the following experiment was performed.

### 5-1. Preparation of artificially contaminated DNA substrate

Artificially contaminated DNA substrates were prepared by adding 100 ng of 0.5 kb uracil-DNA (contaminated DNA, derived from lambda phage) to 100 ng of 1 kb of target DNA (normal DNA, derived from lambda phage). The DNA substrates simulate carryover contamination.

It was investigated whether only the target DNA was selectively amplified when PCR reaction was performed using the artificially contaminated DNA substrate and the UDG of the present invention.

Primers (lambda-1F, lambda-1R) for detecting 0.5 kb uracil-DNA and primers (lambda-2F, lambda-2R) for detecting 1 kb normal DNA were as follows.
lambda-1F: 5'-AATAACGTCGGCAACTTTGG-3' (SEQ ID NO: 9)
lambda-1R: 5'-GTTACGCCACCAGTCATCCT-3' (SEQ ID NO: 10)
lambda-2F: 5'-CAAAGGCGGTTAAGGTGGTA-3' (SEQ ID NO: 11)
lambda-2R: 5'-GGCTGTACCGGACAATGAGT-3' (SEQ ID NO: 12)

### 5-2. Efficacy of Ple UDG in eliminating contaminated uracil-DNA

It was investigated whether the *Ple* UDG of the present invention has the effect of eliminating contaminated uracil-DNA from the artificial contaminated DNA substrate.

In addition to the PCR reagents containing the primers of SEQ ID NOs: 9-12, the *Ple* UDG of the present invention was added to the artificial contaminated DNA substrate as prepared in Example 5-1, in the amount of 0, 1, 2, 3, 4, 5, and 10 ng per 20 µl of the substrate. Thereafter, the mixture was incubated at 25°C for 5 minutes, followed by PCR. The PCR condition was as follows: 94°C for 10 min; 30 cycles consisting of 94°C for 30 sec, 57°C for 30 sec, and 72°C for 90 sec; and 72°C for 5 min. The PCR amplification product was identified by electrophoresis on a 1% agarose gel.

The results are shown in FIG. 8. In FIG. 8, the 1^{st} lane represents a marker DNA, and the 2^{nd} to 8^{th} lanes represent the addition of 0, 1, 2, 3, 4, 5, and 10 ng/20 µl of the *Ple* UDG of the present invention, respectively.

As shown in FIG. 8, when the *Ple* UDG of the present invention was included at 0, 1, 2, 3, or 4 ng/20 µl, the contaminated 0.5 kb uracil-DNA was amplified, whereas when the *Ple* UDG of the present invention was included at 5 or 10 ng/20 µl, the contaminated 0.5 kb uracil-DNA was not amplified but only the 1 kb target DNA was amplified. That is, the UDG of the present invention eliminated the contaminated uracil-DNA depending on the concentration, and the amplification efficiency of the normal 1 kb target DNA also increased as the concentration of the UDG of the present invention increased.

From the above results, it can be seen that the *Ple* UDG derived from *Photobacterium leiognathi* of the present invention has excellent efficacy in eliminating contaminated uracil-DNA at a low temperature of 25°C and excellent amplification efficacy of target DNA because the contaminated DNA is not amplified as a template.

### 5-3. Evaluation of thermal stability of Ple UDG

In order to evaluate the thermal stability of the *Ple* UDG of the present invention, the UDG was first treated at 50°C for varying times (0, 2, 5, 7, 10 minutes), and then added such that 10 ng of UDG is included per 20 µl of the PCR mixture. The artificially contaminated uracil-DNA substrate as prepared in Example 5-1 was used as a substrate, and the PCR mixture was incubated at 25°C for 5 minutes, followed by PCR under the same conditions as in Example 5-2.

The results are shown in FIG. 9. In FIG. 9, the 1^{st} lane represents a marker DNA, the 2^{nd} lane represents a PCR mixture not containing the UDG of the present invention, and the 3^{rd} to 7^{th} lanes represent a PCR mixture containing the UDG of the present invention, which is treated at 50°C for 0, 2, 5, 7 or 10 minutes.

As shown in FIG. 9, when the *Ple* UDG of the present invention was not treated at 50°C, contaminated uracil-DNA was not detected, whereas when the *Ple* UDG of the present invention was treated at 50°C for 2, 5, 7, or 10 minutes, contaminated uracil-DNA was detected. Therefore, it can be seen that the *Ple* UDG of the present invention has low thermal stability such that it can be easily inactivated at 50°C.

### Example 6: Effect of UDG on preventing contamination in real-time PCR

In order to investigate whether the UDG of the present invention has an effect of preventing carryover contamination in a real-time PCR reaction for detecting a plurality of targets in a single tube, the following experiment was performed.

### 6-1. Preparation of artificially contaminated DNA substrate

5 ul of a positive control (PC) of Novaplex^{™} Urinary Tract Infection (UTI) Panel 1 (cat. No. R-SD10228W, Seegene Inc.), a multiplex real-time PCR product capable of detecting a plurality of targets in a single tube, was used as an artificially contaminated DNA substrate. The DNA substrate simulates carryover contamination.

### 6-2. Efficacy of Pie UDG in eliminating contaminated uracil-DNA

It was investigated whether the *Ple* UDG of the present invention has the effect of eliminating contaminated uracil-DNA from the artificially contaminated DNA substrate.

The oligonucleotides of the UTI product were added to the artificially contaminated DNA substrate as prepared in Example 6-1 and the *Ple* UDG of the present invention were added in an amount of 0 and 1 ng per 20 µl, respectively, followed by PCR. The PCR condition was as follows: 95°C for 15 minutes; 45 cycles consisting of 95°C for 3 sec, 60°C for 10 sec, and 72°C for 10 sec. Signals were detected at 60°C and 72°C in each cycle. The Ct values were obtained from the signals detected at the temperatures. The experiment was performed in duplicate.

The results are shown in FIG. 10 and Table 4 below.

The plots of FIG. 10 show the results of detecting the fluorescence signals from fluorophore FAM, HEX, Cal Red 610, Quasar 670, or Quasar 705, after the PCR amplification reaction in the presence (Ple UDG) or absence (No UDG) of 1 ng/20 µl of the *Ple* UDG of the present invention added to various artificially contaminated DNA substrates.

Meanwhile, Table 4 shows Ct values obtained from signals detected in the presence (Ple UDG) or absence (No UDG) of the *Ple* UDG of the present invention.

**<Table 4>**

| Channel | FAM | | HEX | | C610 | | Q670 | | Q750 | |
|---|---|---|---|---|---|---|---|---|---|---|
| Repetition | No UDG | *Ple* UDG | No UDG | *Ple* UDG | No UDG | *Ple* UDG | No UDG | *Ple* UDG | No UDG | *Ple* UDG |
| 1 | 30.37 | 35.14 | 31.69 | NA | 29.21 | 36.70 | 30.32 | 39.57 | 31.36 | NA |
| 2 | 30.50 | 35.06 | 31.84 | NA | 29.25 | 36.16 | 30.20 | 38.21 | 31.29 | NA |

As shown in FIG. 10 and Table 4, it was confirmed that when the *Ple* UDG of the present invention was added, the artificially contaminated DNA substrate was not amplified in the HEX and Q705 channels, and the artificial contaminating DNA substrate was poorly amplified in the FAM, C610, and Q670 channels, compared to when the *Ple* UDG was not added (the Ct value was 5 to 9 higher).

The results show that the *Ple* UDG of the present invention has an excellent effect of eliminating uracil-DNA from a plurality of targets.

Therefore, the use of the *Ple* UDG of the present invention in a PCR process using dUTP instead of dTTP can provide accurate results without carryover contamination of uracil-DNA, demonstrating the usefulness of the *Ple* UDG of the present invention in clinical diagnosis.

## Claims

1. A method for eliminating carryover contamination in a nucleic acid amplification reaction, comprising the step of incubating a composition for the nucleic acid amplification reaction with a sample suspected of containing a nucleic acid, wherein the composition comprises uracil-DNA glycosylase (UDG) derived from *Photobacterium leiognathi.*

2. The method of claim 1, wherein the UDG comprises the amino acid sequence represented by SEQ ID NO: 2.

3. The method of claim 1, wherein the UDG is encoded by the nucleotide sequence represented by SEQ ID NO: 1.

4. The method of claim 1, wherein the incubating is performed at 10 to 25°C.

5. The method of claim 1, wherein the nucleic acid amplification reaction is a polymerase chain reaction (PCR) or an isothermal amplification reaction.

6. A method for producing uracil-DNA glycosylase (UDG) derived from *Photobacterium leiognathi,* comprising the steps of:
(a) culturing a host cell transformed to express UDG derived from *Photobacterium leiognathi;*
(b) obtaining the UDG in the form of inclusion bodies from the cultured host cell;
(c) refolding the resulting UDG in the form of inclusion bodies; and
(d) purifying the refolded UDG.

7. The method of claim 6, wherein the UDG comprises the amino acid sequence represented by SEQ ID NO: 2.

8. The method of claim 6, wherein the UDG is encoded by the nucleotide sequence represented by SEQ ID NO: 1.

9. The method of claim 6, wherein the host cell is *Escherichia coli.*

10. The method of claim 6, wherein the host cell is transformed with an expression vector comprising the nucleotide sequence represented by SEQ ID NO: 1.

11. The method of claim 10, wherein the expression vector comprises a T7 promoter.

12. The method of claim 6, wherein the culturing of step (a) is performed in the presence of IPTG.

13. The method of claim 6, wherein the UDG in the form of inclusion bodies in step (a) is obtained by lysing the host cell.

14. The method of claim 6, which further comprises washing the resulting inclusion bodies after step (a).

15. The method of claim 14, wherein the washing is performed using a buffer containing urea, Tris-HCl, NaCl, Triton X-100, or a combination thereof.

16. The method of claim 6, which further comprises solubilizing the resulting inclusion bodies after step (a).

17. The method of claim 16, wherein the solubilizing is performed using a buffer containing Tris-HCl, NaCl, imidazole, guanidine hydrochloride, 2-mercaptoethanol, or a combination thereof.

18. The method of claim 6, wherein the refolding of step (b) is performed on a column in column chromatography.

19. The method of claim 18, wherein a urea gradient from high concentration to low concentration is applied on the column in column chromatography.

20. Uracil-DNA glycosylase (UDG) derived from *Photobacterium leiognathi,* comprising the amino acid sequence represented by SEQ ID NO: 2.

21. The UDG of claim 20, which has optimal activity at 10 to 25°C.

22. The UDG of claim 20, which is inactivated at 50°C or higher.

23. A nucleic acid molecule comprising a nucleotide sequence encoding the UDG of claim 20.

24. The nucleic acid molecule of claim 20, wherein the nucleic acid molecule comprises the nucleotide sequence represented by SEQ ID NO: 1.

25. A recombinant vector comprising the nucleic acid molecule of claim 24.

26. A host cell comprising the recombinant vector of claim 25.

27. A composition for a nucleic acid amplification reaction, which comprises the UDG of claim 20.
